# EUROPEAN PATENT SPECIFICATION

(11) **EP 0 428 668 B1**
(45) Date of publication and mention of the grant of the patent: **12.04.1995**
(21) Application number: 90908332.1
(22) Date of filing: 22.05.1990
(51) Int. Cl.: A61K 31/70, A61K 31/07

(54) **WATER-SOLUBLE VITAMIN A PREPARATIONS FOR APPLICATION TO THE SKIN**
Wasserlösliche Vitamin A-Zubereitungen und deren Verwendung bei der Hautbehandlung
PREPARATIONS DE VITAMINE A SOLUBLES DANS L'EAU S'APPLIQUANT SUR LA PEAU

(30) Priority: 22.05.1989 US 355778
(43) Date of publication of application: 29.05.1991
(73) Proprietor: IOWA STATE UNIVERSITY RESEARCH FOUNDATION, INC., Ames, Iowa 50011 (US)
(72) Inventor: BARUA, Arun, B., Ames, Iowa (US); OLSON, James, A., Ames, IA 50010 (US); GUNNING, Desiree, Ames, IA 50010 (US)
(74) Representative: Grünecker, Kinkeldey, Stockmair & Schwanhäusser Anwaltssozietät
(86) International application number: US9002783
(87) International publication number: WO9014093

(56) References cited:
- WO-A-86/06275
- US-A- 4 214 000
- Biochemical Pharmacology, vol. 43 (4), 919 - 922 (1992)
- The FSAEB Journal, vol. 5, 320 - 325 (1991)
- Teratology, vol. 47, 29 - 36 (1993)
- Chemotherapy, vol. 21, 236 - 247 (1975)
- Journal of the American Acedemy of Dermatology, vol. 27 (6), S34 - S37 (1992)

## Description

### FIELD OF INVENTION

The field of this invention is the topical application of vitamin A and derivatives thereof for treatment of skin conditions such as acne and skin aging.

### BACKGROUND OF INVENTION

Vitamin A and most derivatives thereof having vitamin A activity are water-insoluble, being hydrophobic, lipid-soluble compounds. For example, neither retinoic acid nor retinol are water-soluble. It is known, however, that the glucuronide and glucose derivatives of retinoic acid and retinol are water-soluble. [See, for example, Lippel and Olson, J. Lipid. Res. 9:580-586 (1968); Takabayashi, et al., Chem. Abs. 73:69834z (1970); Barua, et al., Amer. J. Clin. Nutr., 43:481-485 (1986).] Water-soluble glycoside derivatives of vitamin A are described in United States Patent 4,457,918. The retinoic acid glucuronide and retinol glucuronide have been tested for effects in vitro on cultures of HL-60 cells (a continuous human myeloid cell line): Zile, et al., Proc. Nat. Acad. Sci. USA, 84:2208-2212 (1987); and Gallup, et al., First Mid-America Molecular Biol. Colloquium, p. 36, Oct. 1986; and Zile Biol. & Med., 186:269-274 (1987). Under the conditions of the HL-60 system assay, activity similar to retinoic acid and retinol was reported. Both Gallup, et al. and Zile, et al. found that retinoyl β-D-glucuronide (retinoic acid glucuronide) was 50% less cytotoxic to HL-60 cells than all-trans retinoic acid.

The all-trans form of retinoic acid has been named tretinoin. Cream, gel and liquid preparations containing tretinoin have been approved in the United States for treatment of acne. These preparations are being marketed under the trademark "Retin-A" by the Dermatological Division of Ortho Pharmaceutical Corporation, Raritan, New Jersey. Presently available strengths of "Retin-A" on a weight percent basis are 0.025% or 0.01% for the gel, 0.1% or 0.05% for the cream, and 0.05% for the liquid. Other potential uses for topical tretinoin have been reported, including treatment of aging skin (Weiss, et al., J. Amer. Acad. Dermat. 19:169-175, 1988), and treatment of xerophthalmia and corneal epithelial wounds (Ubels, et al., Current Eye Research, 4:1049-1057 1985).

Topical tretinoin is being experimentally tested for treatment of aging skin. It has been found, however, that the patients under treatment may experience local erythema and peeling of the skin, as has been observed in acne treatment. The degree of irritation varies with the strength of the preparation and the frequency of its application, higher concentrations and more frequent application producing a greater number of skin irritation side effects. When such side effects are noted, the treatment can be discontinued for a number of days, a preparation of lower strength used, or the time between applications can be increased. In view of the clinically beneficial results obtained in the treatment of acne and the promising results with respect to improving the condition of aging skin, it would be desirable to utilize a less irritating form of retinoic acid.

Weiss, et al. (1988), cited above, reported a double-blind study confirming earlier published reports that treatment with tretinoin is capable of at least partly reversing structural damage associated with extrinsic aging (photoaging) of the skin. In order to minimize skin irritation, Weiss et al. started treatment with 0.1% tretinoin cream on a daily basis, and then advanced the patients to twice-a-day applications. When a patient was unable to tolerate the 0.1% cream, the patient was switched to a 0.05% cream. Most patients were able to continue with the treatments. However, during the course of their study Weiss, et al. found that "more than 90% of the patients experienced some degree of dermatitis in the tretinoin treated areas" (page 172, col. 1). The areas of the skin found most susceptible to retinoid dermatitis were the sides of the neck, the V-area of the neck and chest, the ventral arms, and the antecubital fossae.

### SUMMARY OF INVENTION

This invention in general relates to topical medicaments for application to the epithelium. In specific embodiments, it relates to topical medicaments for application to the skin for treatment of acne and/or skin aging. The topical medicaments of this invention use a specific water-soluble vitamin A derivative in admixture with spreadable topical carriers, such as gels, creams, liquid preparations such as lotions. The active ingredient of the medicaments is retinoic acid glucuronide. This compound is preferably in all-trans form, but it may be used in its 13-cis form. The topical medicaments of this invention preferably utilize carriers which contain an aqueous or polar solvent phase so that the water-soluble vitamin A derivative can be dissolved therein.

The preparations of this invention are much less irritating to the skin than retinoic acid preparations. Not only does the glucuronide moiety provide water-solubility, but it also reduces side effects resulting in skin redness, scaling, and the like. Since the preparations of this invention are appreciably less irritating to the skin than the tretinoin preparations currently in use, they can be used at higher concentrations and/or with more frequent applications. This can increase the effectiveness of treatments for acne and skin aging.

### DETAILED DESCRIPTION

The water-soluble vitamin A derivative used in the preparations of this invention are not available commercially, but they can be prepared from commercially available starting materials. These materials include principally retinoic acid which can be obtained from commercial sources in all-trans or 13-cis forms, for example, from Sigma Chemical Co., St. Louis, MO, U.S.A. The compounds all-trans and 13-cis retinoic acid glucuronide can be prepared by known reactions.

Retinoyl fluoride is a useful intermediate for preparing retinoic acid glucuronide also called retinoyl β-glucuronide. A method for preparing all-trans or 13-cis retinoyl fluoride is described in Barua and Olson, Biochimica. et Biophysica Acta, 757:288-299 (1983); and U.S. Patent 4,473,503. A two-step process for preparing retinoic acid glucuronide from retinoyl fluoride is described in Barua and Olson, J. Lipid. Res., 26:1277-1282 (1985). Retinoyl fluoride is first reacted with 6,3-glucuronolactone to produce 6,3-lactone of retinoyl glucuronic acid, which is then hydrolyzed with dilute alkali to give the retinoyl β-glucuronide. A one-step process for this synthesis is described in United States patent 4,855,463. The reaction is carried out in an acetone-water mixture proportioned so that both the water-insoluble retinoyl fluoride and the water-soluble glucuronic acid are maintained in solution.

The preparations of this invention can be formulated as ointments or lotions. The preparations preferably contain a polar or aqueous phase in which the water-soluble vitamin A derivative is dissolved. For example, aqueous gels can be used as an ointment base, and liquid preparations can be formulated as aqueous solutions, or with a polar solvent such as glycerol. Alternatively, however, an ointment can be formed from a lipid-base cream. The vitamin A compounds are soluble in polar organic solvents, such as glycerol, and can be formulated therein. Glycerol solutions of the compounds can be combined with gels, creams, or liquid preparations.

Suitable water-soluble ointment bases include polyethylene glycol. Emollients may be included and also preservatives, such as the parabens. More complex topical carrier formulations can also be used. For example, a gel vehicle may consist of butylated hydroxytoluene, hydroxypropyl cellulose, and ethanol. A cream vehicle may consist of a mixture of stearic acid, isopropyl myristate, polyoxyl-stearate, stearyl alcohol, xanthan gum, sorbic acid, butylated hydroxytoluene, and water. A liquid vehicle may be composed of a mixture of polyethylene glycol, butylated hydroxytoluene, and ethanol. A topical vehicle for tretinoin and isotretinoin is described in United States Patent 4,727,088. This vehicle, which contains long chain fatty alcohols and a volatile silicone, can be used with the water-soluble derivatives of this invention.

Topical medicaments of this invention and the described vitamin A derivatives (all-trans and 13-cis retinoic acid glucuronide) can be combined with the topical carrier (ointment or liquid) in a wider range of strengths than has heretofore been available for retinoic acid preparations. For example, the preparations may be formulated to contain from 0.02 to 0.5 weight percent of the active water-soluble retinoid. On the basis of present information, it is believed that the most useful formulations will contain from 0.05 to 0.35% by weight of retinoic acid glucuronide, preferably in all-trans form. To maximize therapeutic effect, the preparations may need to contain more than 0.15% of the active retinoid, such as 0.2% up to 0.5% by weight.

It will be understood that the retinoic acid glucuronide should be homogeneously dispersed in the topical preparations, such as by dissolving in the aqueous phase or by thorough mixing with the vehicle if it does not contain an aqueous or polar phase. For example, a finely divided powder of the compound can be dispersed in an oil-based vehicle such as petrolatum.

The preparations and method of this invention are further illustrated by the following examples.

### EXAMPLE I

### Synthesis of Preferred Retinoyl β-Glucuronide (Retinoic Acid Glucuronide)

Retinoyl fluoride (2.4 g, 7.9 mmol) was dissolved in 200 ml of acetone. Preferably all-trans retinoyl fluoride is used but the reaction is the same for 13-cis retinoyl fluoride. D-glucuronic acid (6 g, 31 mmol) is dissolved in 50 ml of water and sodium bicarbonate (970 mg) dissolved in 50 ml of water were added to the retinoyl fluoride solution. The mixture was stirred at room temperature for 20-24 h. The solution was neutralized with 1N HC1, diluted with water, and the product was extracted with ethyl acetate. The extract was washed with water, dried over anhydrous sodium sulfate and then evaporated to dryness in a rotary evaporator. The residue was dissolved in 2-3 ml of diethyl ether and transferred to a silica gel (for dry column chromatography, wet packed with hexane) column. The column was developed with hexane containing diethyl ether (5-50%) to remove retinoic acid and other products. The major yellow band containing retinoyl glucuronide was next eluted with a mixture of CH₂Cl₂/CH₃PH (1:1). The solvent was evaporated to dryness to give solid retinoyl β-glucuronide (2.3 g, 60%). This preparation consisted mainly of the all-trans isomer, and can be used as such.

Analytically pure retinoyl β-glucuronide (retinoic acid glucuronide) was obtained by HPLC of the above preparation on a Whatman ODS-3 column (M9, 50 cm) using methanol/water (7:3) containing 10mM ammonium acetate at a flow rate of 3 ml/min. Retinoyl β-glucuronide (t_{R}=48.7 min) separated from traces of isomers or anomers (t_{R}=46.4 min). The eluate carrying retinoyl β-glucuronide was diluted with water, made just acidic with 0.1N HC1 and the product extracted with ethyl acetate. The extract was washed with water, dried over anhydrous sodium sulfate and then evaporated to dryness. The residue was dissolved in a small volume of diethyl ether and all-trans retinoyl β-glucuronide was precipitated with hexane. The solid was separated and dried. All-trans retinoyl β-glucuronide:m.p. 142-143°C (darkens at 125°C); UVₘₐₓ360 nm (E_{1cm} = 1065) in methanol and 365 nm in water. The ¹H-NMR, IR and Mass spectra and C, H analysis results were consistent with the structure. The compound is soluble in water. Incubation of retinoyl β-glucuronide with β-glucuronidase (from E. coli) in phosphate buffer (pH 6.8) for 0.5-2 h generated retinoic acid.

### EXAMPLE II

### Preparation of Retinoic Acid Glucuronide Cream

Fifty mg of finely powdered all-trans retinoic acid glucuronide (retinoyl β-glucuronide) was dissolved in 2 g of glycerol by agitating the mixture for several minutes. A commercial lotion (Nivea®) (48 g) was added to the solution to bring the concentration of the retinoid to 0.1%. The mixture was stirred well and stored protected from light and heat. Nivea® moisturizing lotion is manufactured by Beiersdorf, Inc., Norwalk, CT), and is composed of water, mineral oil, isohexadecane, petrolatum, sorbitol, PEG-40, sorbitol peroleate, lanolin acid glycerin ester, propylene glycol, cetyl palmitate, tromethamine, magnesium aluminium silicate, magnesium sulfate, fragrance, aluminium stearate, lanolin alcohol, BHT, methylchloroisothiazolinone and methylisothiazolinone.

Another preparation was made in the same way adding 125 mg of the retinoid to 3 g of glycerol and 47 g of the Nivea® lotion to give a concentration of 0.25%.

For comparison a retinoic acid cream was prepared as follows: 50 mg of solid all-trans retinoic acid was dissolved in glycerol (2 g) and mixed with 48 g of Nivea® cream to give a concentration of 0.1%.

### EXAMPLE III

### Preparation of Retinoic Acid Glucuronide Lotion

Fifty mg of finely powdered retinoic acid glucuronide was dissolved in 10 g of glycerol by agitating for several minutes. The solution was diluted with 40 ml of water to give a 0.1% lotion of retinoid glucuronide.

### EXAMPLE IV

### Treatment of Face for Acne

A female volunteer with facial acne applied the 0.1% retinoic acid glucuronide preparation of Example II on her right face, and 0.1% retinoic acid cream prepared in the same way on her left face every night before going to bed.

Within two weeks, the left side (retinoic acid treatment) became tender and red, and peeling of skin occurred. Facial washing and application of make-up became extremely painful. The burning and degree of discomfort forced her to discontinue the treatment with retinoic acid.

The right side of the face (glucuronide treatment) did not show any sign of the above problems, although at the beginning the eyelids were highly sensitive to the cream, which sensitivity disappeared within a few days. After a month of the glucuronide treatment, the face was clear and smooth with no visible blemishes.

Application of the 0.25% glucuronide preparation of Example II on the volunteer's face did not result in any burning or peeling or other discomfort.

Another female volunteer with facial acne has been treated with the 0.1% glucuronide preparation of Example II. A marked degree of progress has been observed in a few weeks. No burning or peeling of skin or other discomforts have been noted.

### EXAMPLE V

### Treatment for Wrinkles

A male volunteer with slight wrinkles around his eyes applied 0.1% lotion of the retinoic acid glucuronide lotion of Example III on his face every night for over six months. The severity of wrinkles diminished considerably. No side effects were observed or reported.

### EXAMPLE VI

### Treatment for Cracked Lips

During winter and dry season, when his lips were cracked, a male volunteer applied the 0.1% retinoic acid glucuronide lotion of Example III on his lips before going to bed. The cracks disappeared with a few days of application.

## Claims

1. The use of retinoic acid glucuronide in an all-trans or 13-cis form for the preparation of a medicament for the treatment of skin acne, skin wrinkles, and/or lip cracks.

2. The use according to claim 1 wherein the retinoic acid glucuronide is in admixture with a topical carrier selected from gels, creams, ointments and lotions.

3. The use according to claim 1 wherein the retinoic medicament provides an aqueous phase.

4. The use according to claims 1, 2 or 3 wherein the retinoic acid glucuronide is present in an amount of from 0.02 to 0.5 weight percent.

5. The use according to claim 1 wherein the retinoic acid glucuronide is in all-trans form.

## Patentansprüche

1. Verwendung von Retinoesäureglucuronid in vollständiger trans- oder 13-cis-Form für die Herstellung eines Medikaments für die Behandlung von Hautakne, Hautfalten und/oder Lippenrissen.

2. Verwendung nach Anspruch 1, wobei das Retinoesäureglucuronid mit einem topischen Träger, ausgewählt aus Gelen, Cremen, Salben und Lotionen, vermischt ist.

3. Verwendung nach Anspruch 1, wobei das retinoische Medikament eine wäßrige Phase bereitstellt.

4. Verwendung nach Anspruch 1, 2 oder 3, wobei das Retinoesäureglucuronid in einer Menge von 0,02 bis 0,5 Gew.-% anwesend ist.

5. Verwendung nach Anspruch 1, wobei das Retinoesäureglucuronid in vollständiger trans-Form ist.

## Revendications

1. Utilisation de glucuronide de l'acide rétinoïque dans une forme toute *trans* ou 13-*cis* pour la préparation d'un médicament pour le traitement de l'acné cutané, des rides cutanées et/ou des gerçures des lèvres.

2. Utilisation selon la revendication 1, dans laquelle le glucuronide de l'acide rétinoïque est mélangé avec un support topique choisi parmi les gels, les crèmes, les pommades et les lotions.

3. Utilisation selon la revendication 1, dans laquelle le médicament rétinoïque fournit une phase aqueuse.

4. Utilisation selon les revendications 1, 2 ou 3, dans laquelle le glucuronide de l'acide rétinoïque est présent en une quantité de 0,02 à 0,5 % en poids.

5. Utilisation selon la revendication 1, dans laquelle le glucuronide de l'acide rétinoïque est dans une forme toute *trans*.
